# EUROPEAN PATENT APPLICATION

(11) **EP 1 547 562 A1**
(43) Date of publication of application: **29.06.2005**
(21) Application number: 03797528.1
(22) Date of filing: 26.08.2003
(51) Int. Cl.: A61F 13/514

(54) **ILLUSTRATED DISPOSABLE DIAPER**

(30) Priority: 18.09.2002 JP 2002272317
(71) Applicant: UNI-CHARM CORPORATION, Kawanoe-shi Ehime 799-0111 (JP)
(72) Inventor: SUZUKI, Sachiyo Technical Center, Uni-Charm Corp., Mitoyo-gun, Kagawa 769-1602 (JP); ISHIKAWA, Hiroki Technical Center, Uni-Charm Corp., Mitoyo-gun, Kagawa 769-1602 (JP); SAKAGUCHI, Satoru Technical Center,Uni-Charm Corp., Mitoyo-gun, Kagawa 769-1602 (JP); TANGE, Akiko Technical Center, Uni-Charm Corp., Mitoyo-gun, Kagawa 769-1602 (JP); MIYAWAKI, Masami Technical Center, Uni-Charm Corp., mitoyo-gun, Kagawa 769-1602 (JP)
(74) Representative: Staeger - Sperling
(86) International application number: PCT/JP2003/010768
(87) International publication number: WO 2004/026205

(57) **Abstract**

The present invention relates to a disposable diaper with one or more illustrations. The disposable diaper is constituted of such illustrations that the illustrations can be recognized clearly from outside if viewed in one direction even when the disposable diaper is in a folded state after being taken out from a package by a diaper exchanger. Thus, the illustrations may be fully utilized.

## Description

### FIELD OF THE INVENTION

The present invention relates to a disposable diaper with an illustration, and more specifically it relates to a folded disposable diaper with an illustration.

### BACKGROUND ART

As a conventional disposable diaper, a type of disposable diaper is known which has left and right side edges at the ventral potion thereof and left and right side edges at the dorsal portion thereof such that left side edges of the ventral and dorsal portions are joined each other and that right side edges of the ventral and dorsal portions are joined each other so as to form a single waist opening and a pair of left and right leg openings. This type of disposable diaper has an absorbent body positioned at least at a groin portion or vicinity thereof. Recently, in order to meet a taste of a mother or other guardians and improve a design effect, relatively large illustrations of animals, animation characters, etc., are printed on such disposable diapers, especially on the outer face of the dorsal portion and/or ventral portion of disposable diapers for infants. By printing an illustration, etc., on an outer face of a disposable diaper, the illustration, etc., can be perceived or viewed from the outside of the diaper. Also, such an illustrated disposable diaper has a folded tape for waste disposal purposes positioned on the outer surface as a waste disposal means. Though such a disposable diaper with an illustration is generally not folded in particular, recently in order to improve portability by folding compactly or to improve the packaged form and appearance at a store, various ways of folding have been proposed (Japanese UtilityModel Publication No. Hei 7-22256 and Japanese Unexamined Patent Publication No. 2000-116704).

However, despite such efforts, such measures were not favored in particular by diaper exchangers who exchange illustrated disposable diapers and infants who are the wearers of the diapers and did not provide adequate appeal.

### SUMMARY OF THE INVENTION

In an aspect of the present invention, it is an object to provide a disposable diaper with an illustration provided in a way that the effects of the illustration can be exerted adequately.

Upon carrying out a diligent research , the inventors found that the mutual relationship between the diaper exchanger and the infant before exchanging the diaper is important such that the infant would not be fussy about exchanging diapers and the exchanger can exchange diapers pleasantly.

The establishment of good mutual communication is a key point in maintaining the mutual relationship between the exchanger and the infant, and the illustration of a character figure, etc., printed on the disposable diaper can be used by the exchanger so that the exchanger can open up the communications with the infant.

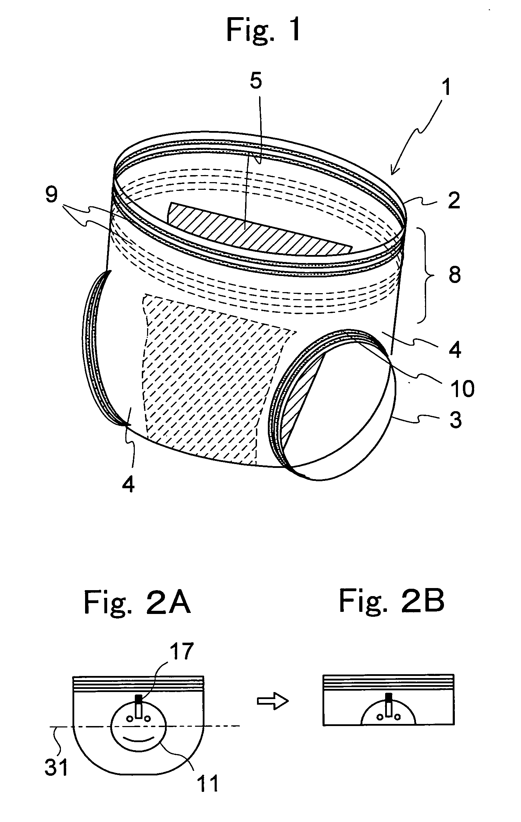

Figs. 2A and 2B show a conventional disposable diaper with an illustration. When this diaper is folded along a crease 31 in a similar way of folding as in the process of selling the diaper, the illustration is also folded, and thus, the illustration is divided by the crease. Thus, when the folded disposable diaper is viewed from one direction, it would be difficult to tell what illustration is drawn so that the design quality of the disposable diaper may be impaired. Also, the illustration may not serve as an opportunity for the starting of communication between the diaper exchanger and the infant and becomes useless as a means of maintaining a mutual relationship. The design quality of the disposable diaper with the illustration is also impaired if a tape or other waste disposal means for the illustrated disposable diaper is attached over or near the illustration on the outer face of the disposable diaper. Furthermore in this case, the illustration becomes hard to discern and the mutual relationship between the exchanger and the infant is also affected.

Thus, according to the present invention, a disposable diaper with one or more illustrations is arranged so that at least one illustration can be recognized clearly at one glance from the outside even when the disposable diaper is folded just after it is taken out of its package by the diaper exchanger.

The design quality of the disposable diaper in the folded state immediately after being taken out from the package is thus improved, the communication between the infant and the diaper exchanger by means of the illustration is improved, and yet the disposable diaper can be folded compactly.

The present invention has been made as a result of diligent research of the mutual relationship between the infant and the diaper exchanger in and before a diaper exchange period.

More specifically, the following is provided according to the present invention.
(1) A disposable diaper with illustrations comprising an illustration display portion; wherein the illustration display portion comprises a plurality of illustration display sub-portions on which the illustrations are displayed; wherein the illustration display sub-portions are provided on a plurality of display faces of the disposable diaper respectively; and wherein at least a main portion of one of the illustrations is provided at a position such that the main portion is visually recognizable from an outside when the disposable diaper is folded in a way of folding so as to be contained in a compact bag and/or to be displayed for sale.
   In an aspect of the present invention, an illustration on the disposable diaper could be identified even when the disposable diaper is folded immediately after it is taken out form a package thereof. The identified illustration improves communications by means between the infant and the diaper exchanger, lessens uncomfortableness of the infant during the diaper exchange, and facilitates diaper exchanging pleasantly.
   Here, that the main portion of the illustration is visually recognizable means that an integral part (main portion) that characterizes the contents of the illustration is shown in a manner that the integral part is not divided by a folding line, which comprises a crease, of the disposable diaper in the folded state. Thus, the illustration can be identified as long as what the illustration expresses is understandable although a missing part of the illustration is off from a visually recognizable position due to the way of folding the disposable diaper. The missing part of the illustration may be positioned on another face of the disposable diaper or may be hidden inside. For example, suppose the illustration is comprised of a fisherman who is dangling a fishing line. Even if the disposable diaper is folded in the middle of a long and dangling fishing line, a person may not be prevented from comprehending that the illustration shows a fisherman dangling a fishing line.
(2) A disposable diaper with illustrations comprising an illustration display portion; wherein the illustration display portion comprises a plurality of illustration display sub-portions on which different illustrations are displayed, respectively; wherein the illustration display sub-portions are respectively provided at a plurality of display faces of the disposable diaper; and wherein at least one illustration among the illustrations is not divided by being folded, the illustrations being provided at visually recognizable positions from an outside when the disposable diaper is folded in the way of folding so as to be contained in a compact bag and/or to be displayed for sale.
   According to the present invention, in the disposable diaper with illustrations, at least one illustration appears in an undivided manner so as to be identified even when the disposable diaper is in the folded state after being taken out from the package. Thus, good communications by means of the illustration may be enabled between the infant and the diaper exchanger.
   Here, that an illustration drawn on a disposable diaper is divided refers, for example, to a case where a part of the illustration appears on one face of the disposable diaper and another part of the illustration appears on another face of the disposable diaper due to a way of folding. That an illustration is divided also refers, for example to, a case where a part of the illustration is folded such that the part is positioned between faces contacting each other of the disposable diaper resulting in missing the part from the entire illustration if viewed from the outside.
(3) The disposable diaper according to (2) is characterized in that none of the illustrations is divided by being folded, the illustrations being provided at visually recognizable positions from an outside when the disposable diaper is folded in the way of folding so as to be contained in a compact bag and/or to be displayed for sale.
   According to the present invention, since all illustrations, which are positioned such that they can be viewed from the outside in the folded state, appear in an undivided manner, the disposable diaper with the illustrations has high design quality.
(4) The disposable diaper according to any from (1) to (3) is characterized in that the disposable diaper is folded in a way of folding such that first and second illustrations among the illustrations such that the folded disposable diaper has two outside faces and the first and second illustrations are provided on the two outside faces, respectively.
   According to the present invention, since illustrations are positioned on two outside faces of the disposable diaper in the folded state, at least one illustration can be seen when the folded disposable diaper is placed. Also, suppose that illustrations having different contents are positioned on the respective faces of the disposable diaper, the diaper exchanger can show the infant the illustrations on the faces of the folded disposable diaper in a turning manner and have the infant enjoy the different illustrations. Therefore, good communications by means of the illustrations can be made between the infant and the diaper exchanger.
(5) The disposable diaper according to any from (1) to (4) further comprises another illustration provided at an inside face of folded portions of the disposable diaper being provided when the disposable diaper is folded in the way of folding so as to be contained in a compact bag and/or to be displayed for sale.
   According to the present invention, the illustrations may also be positioned on inner faces when the disposable diaper is folded. When illustrations drawn on faces of the dorsal side and the ventral side, the diaper exchanger can first show some of the illustrations on either face being on the outside of the folded disposable diaper to the infant and then unfold the folded disposable diaper so as to show some of the illustrations positioned on either face having been on the inside or having being an inner face. Thus, the diaper exchanger may have the infant enjoy a variety of illustrations and further have good communications with the infant from when the folded disposable diaper is unfolded to when the exchanged diaper is put on the infant.
(6) The disposable diaper according to any from (1) to (5) is characterized in that yet another illustration among the illustrations is completed within any one of outside faces of the folded disposable diaper, the yet another illustration being provided at a visually recognizable position from an outside when the disposable diaper is folded in the way of folding so as to be contained in a compact bag and/or to be displayed for sale.
(7) The disposable diaper according to any from (1) to (6) is characterized in that each illustration among the illustrations has a feature of a story, the each illustration being provided at respective outside faces of the disposable diaper when the disposable diaper is folded in the way of folding so as to be contained in a compact bag and/or to be displayed for sale.
   According to the present invention, since the illustrations positioned at both faces of the folded disposable diaper have the feature of the story, the exchanger of the disposable diaper can show the faces of the folded disposable diaper in a turning manner to talk to the infant about the development of the story. An opportunity for good communications with the infant, who is several months old and is in a stage of developing intellectual faculties, is thus provided.
(8) The disposable diaper according to any from (1) to (7) comprises means for disposing provided on an outer surface of the disposable diaper, wherein the disposable diaper is folded such that the means for disposing is hidden when the disposable diaper is folded in the way of folding so as to be contained in a compact bag and/or to be displayed for sale.
   Here, the means for disposing refers to means for rounding up and stopping the disposable diaper for waste disposal after use. Since the means for disposing is hidden and not visible in the state that the disposable diaper is folded, the aesthetic appearance of the disposable diaper can be maintained. Also, the eyes of one who sees the disposable diaper can be made to focus more on the illustrations.
(9) The disposable diaper according to (8) is characterized in that the means for disposing is provided off a crease of the disposable diaper and the illustrations.
(10) The disposable diaper according to (8) or (9) is characterized in that the means for disposing comprises a folded tape.
(11) The disposable diaper according to any from (1) to (10) is characterized in that the disposable diaper is formed as a pants type diaper.
(12) The disposable diaper according to any from (1) to (11) comprises a ventral portion that is positioned at a ventral side of a wearer when worn, a dorsal portion that is positioned at a dorsal side of the wearer when worn, a pair of left and right leg openings at the side edges of the ventral and dorsal portions, and a waist opening at the upper edges of the ventral and dorsal portions.
(13) A disposable diaper with an illustration comprises a crease along which the disposable diaper is folded, wherein the illustration is substantially provided off the crease.
   Here, that the illustration is provided off the crease means that no crease is substantially provided on the illustration such that the illustration is substantially fee of crease.
   According to the present invention, even when the disposable diaper is folded at one or more creases, the illustration will not be divided by the creases and the illustration can be displayed as it is. That the illustration is substantially free of crease means that there are practically or substantially no creases on the illustration. Also, being practically free of crease may refer to a case that absolutely no crease is provided on the illustration as well as that some creases are provided at end portions of the illustration such that the illustration may be recognized visually.
(14) The disposable diaper according to (13) is characterized in that the illustration and the crease are provided in a substantially not overlapping manner.
   According to the present invention, even when the disposable diaper is folded along the crease, the illustration is not divided by the crease and the illustration can be displayed as it is.

### BRIEF DESCRIPTION OF THE DRAWINGS

Fig. 1 is a perspective view showing the overall arrangement of an illustrated disposable diaper according to an embodiment of the present invention.
Fig. 2A is a rear view showing a conventional disposable diaper.
Fig. 2B is a rear view showing the conventional disposable diaper when it is folded at a crease.
Fig. 3A is a front view showing a disposable diaper with illustrations according to an embodiment of the present invention.
Fig. 3B is a rear view of the disposable diaper in Fig 3A.
Fig. 3C is a plan view of the disposable diaper in Fig 3A that is folded along creases.
Fig. 3D is a front view of the disposable diaper in Fig 3A that is folded along the creases.
Fig. 3E is a side view of the disposable diaper in Fig 3D.
Fig. 3F is a front view of the disposable diaper in Fig. 3E that is folded along creases.
Fig. 3G is a rear view of the disposable diaper in Fig. 3F.
Fig. 3H is a side view of the disposable diaper in Fig. 3F.
Fig. 4A is a front view showing a disposable diaper with illustrations according to another embodiment of the present invention.
Fig. 4B is a rear view of the disposable diaper in Fig 4A.
Fig. 4C is a plan view of the disposable diaper in Fig 4A that is folded along creases.
Fig. 4D is a front view of the disposable diaper in Fig 4A that is folded along the creases.
Fig. 4E is a side view of the disposable diaper in Fig 4D.
Fig. 4F is a front view of the disposable diaper in Fig. 4E that is folded along creases.
Fig. 4G is a side view of the disposable diaper in Fig. 4F.
Fig. 5A is a front view showing a disposable diaper with illustrations according to yet another embodiment of the present invention.
Fig. 5B is a rear view of the disposable diaper in Fig 5A.
Fig. 5C is a plan view of the disposable diaper in Fig 5A that is folded along creases.
Fig. 5D is a front view of the disposable diaper in Fig 5A that is folded along the creases.
Fig. 5E is a side view of the disposable diaper in Fig 5D.
Fig. 5F is a front view of the disposable diaper in Fig. 5E that is folded along creases.
Fig. 5G is a side view of the disposable diaper in Fig. 5F.
Fig. 6A is a front view showing a disposable diaper with illustrations according to another embodiment of the present invention.
Fig. 6B is a rear view of the disposable diaper in Fig 6A.
Fig. 6C is a plan view of the disposable diaper in Fig 6A that is folded along creases.
Fig. 6D is a front view of the disposable diaper in Fig 6A that is folded along creases.
Fig. 6E is a side view of the disposable diaper in Fig 6D.
Fig. 6F is a front view of the disposable diaper in Fig. 6E that is folded along a crease.
Fig. 6G is a side view of the disposable diaper in Fig. 6F.
Fig. 7A is a front view showing a disposable diaper with illustrations according to another embodiment of the present invention.
Fig. 7B is a rear view of the disposable diaper in Fig 7A.
Fig. 7C is a plan view of the disposable diaper in Fig 7A that is folded along a crease.
Fig. 7D is a front view of the disposable diaper in Fig 7A that is folded along the crease.
Fig. 7E is a front view of the disposable diaper in Fig 7D that is folded along a crease.
Fig. 8A is a front view showing a disposable diaper with illustrations according to another embodiment of the present invention.
Fig. 8B is a rear view of the disposable diaper in Fig 8A.
Fig. 8C is a front view of the disposable diaper in Fig 8A that is folded along a crease.
Fig. 8D is a side view of the disposable diaper in Fig. 8A.
Fig. 8E is a sid view of the disposable diaper in Fig 8C.
Fig. 9A is a front view showing a disposable diaper with illustrations according to an embodiment of the present invention.
Fig. 9B is a rear view of the disposable diaper in Fig 9A.
Fig. 9C is a plan view of the disposable diaper in Fig 9A that is folded along creases.
Fig. 9D is a front view of the disposable diaper in Fig 9A that is folded along the creases.
Fig. 9E is a side view of the disposable diaper in Fig 9D.
Fig. 9F is a front view of the disposable diaper in Fig. 9E that is folded along creases.
Fig. 9G is a rear view of the disposable diaper in Fig. 9F.
Fig. 9H is a side view of the disposable diaper in Fig. 9F.
Fig. 10A is a front view showing a disposable diaper with illustrations according to an embodiment of the present invention.
Fig. 10B is a rear view of the disposable diaper in Fig 10A.
Fig. 10C is a plan view of the disposable diaper in Fig 10A that is folded along creases.
Fig. 10D is a front view of the disposable diaper in Fig 10A that is folded along the creases.
Fig. 10E is a side view of the disposable diaper in Fig 10D.
Fig. 10F is a front view of the disposable diaper in Fig. 10E that is folded along creases.
Fig. 10G is a rear view of the disposable diaper in Fig. 10F.
Fig. 10H is a side view of the disposable diaper in Fig. 10F.

### DETAILED DESCRIPTION OF PREFERRED EMBODIMENTS

Fig. 1 is a perspective view of a disposable diaper according to an embodiment of the present invention. However, the disposable diaper according to the present invention is limited to the embodiments described in the specification.

A disposable diaper 1, which is shown in Fig. 1, comprises an outer part 4 which has a waist opening 2 and a pair of leg openings 3 formed therein, and an inner part which is provided at a groin portion and a vicinity thereof of the outer part 4. The disposable diaper 1 can be formed by fixing the inner part to the outer part 4. In putting the outer part 4 and the inner part together, they may be bonded with heat sealing, ultrasonic sealing, hot-melt adhesive, etc.

The inner part comprises a top sheet 5 being provided at askin-contactingsurface, and an absorbent body being positioned at an interior part formed by the top sheet 5 and the outer part 4. A hydrophilic nonwoven fabric or a liquid-permeable sheet made of porous plastics, etc., is used as the top sheet. Here, a nonwoven fabric refers to a fabric prepared with the spun lace, span bond, needle punch, melt-blown, thermal bond, chemical bond, air-through, etc. As the fibers of the nonwoven fabric, polyolefin-, polyester-, or polyamide-based fibers or sheath-core type composite fibers or side-by-side fibers formed of polyethylene, polypropylene, or polyester may be used.

The absorbent body is composed of hydrophilic fibers and super absorbent polymer. As the hydrophilic fibers, absorbent fibers such as pulp, rayon, acetate, cotton, etc., fibers prepared by making synthetic thermoplastic resin fibers hydrophilic, and so on may be employed. A plastic film may also be provided to prevent leakage of the retained body fluids.

In order to realize resistance against fluid, pleasant texture, and air permeability at the same time, the outer part 4 is composed of a single sheet or a plurality of sheets. For example, a hydrophobic nonwoven fabric, a water-impermeable plastic film, or a sheet formed by laminating such fabric and/or film may be used. The above-mentioned plastic film can be improved in the air permeability and moisture permeability by stretching after mixing a filler.

At a waist portion 8 of the disposable diaper 1, a plurality of elastic members 9, which stretch along the waist opening 2, are provided. A plurality of elastic members 10, which stretch along leg openings 3 are also provided. These elastic members 9 and 10 may be provided between a plurality of sheets that constitute the outer part 4 or may be provided between two sheets in a stretched manner and bonded with the hot melt.

The elastic members 9 and 10 may comprises a plastic sheet made of natural rubber, synthetic rubber, or synthetic thermoplastic resin.

At least one or more illustrations of a specific person, animal, plant, or other character figures are positioned at a portion or portions of the dorsal side or ventral side of the outer part 4 that can be viewed from the outside. The illustrations drawn on the disposable diaper may be positioned on either inner or outer part as long as the illustration can be recognized when viewed from the outside of the disposable diaper according to the present invention. The illustrations may be printed directly on the sheets of the disposable diaper or other sheets which are adhered to the disposable diaper later. For example, in a case that the illustrations are to be positioned on the inner part side, a film onto which an illustration is printed may be adhered onto the lowermost layer (i. e., the layer closest to the outer part) to enable the illustration to be seen through the outer part 4. In a case that an illustration is to be positioned on the outer part side, the illustration may be printed directly onto an outermost nonwoven fabric layer of the outer part or may be printed onto a film, over which a nonwoven fabric is placed so that the illustration can be seen through the nonwoven fabric from the outside.

Figs. 2A and 2B show an arrangement of the conventional disposable diaper with the illustration. Figs. 2A and 2B show the dorsal side of the disposable diaper. In Fig. 2A, the illustrationof a character figure is positioned at substantially the center of the dorsal side of the disposable diaper.

Here, during the stages of distribution and selling, the disposable diaper is packaged, not in a spread state as shown in Fig. 2A, but in a folded state as shown Fig. 2B in a package. In this case, the illustration that is positioned at the center in the front view of Fig. 2A of the disposable diaper is divided along a crease 31 so that one part thereof appears on one outer face of the disposable diaper having been folded into two and the other part appears on the other outer face.

Thus the disposable diaper having been taken out from a package thereof by the diaper exchanger is in the folded state and the exchanger cannot recognize what character figure the illustration expresses by glancing at the folded disposable diaper from one side. The infant (i.e., wearer) also cannot recognize what character figure the illustration expresses when the folded disposable diaper is shown.

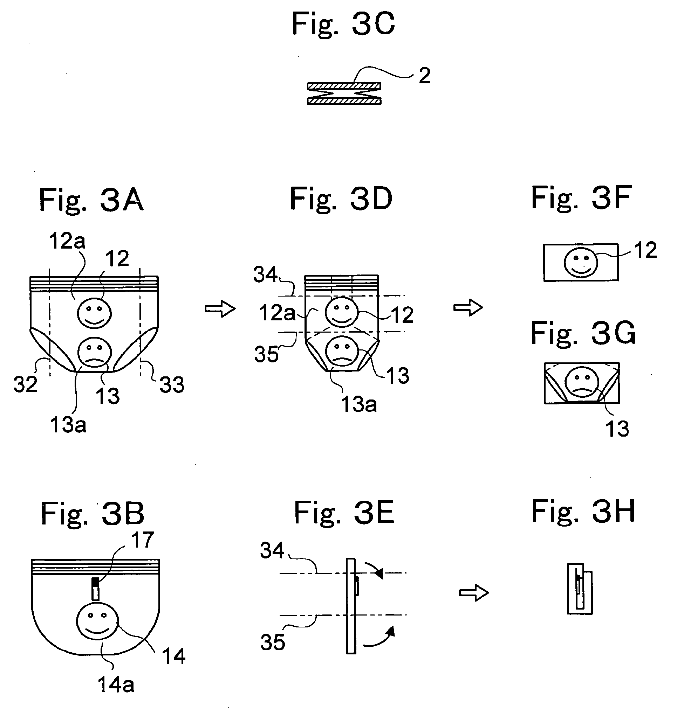

Figs. 3A through 3H show an embodiment of an disposable diaper with illustrations as an embodiment of the present invention. Fig. 3A shows the ventral side of the disposable diaper and Fig. 3B shows the dorsal side of the disposable diaper. An illustration 14, which is positioned at a illustration display sub-portion 14a on the dorsal side, is positioned such that the illustration 14 does not overlap a waste disposing tape 17. Illustrations 12, 13 are positioned on illustration display sub-portions 12a, 13a on the ventral side as well as on the dorsal side. The illustrations 12 and 13, which are positioned at the illustration display sub-portions 12a, 13a on the ventral side, are separately located on the waist opening side and the groin portion side. The illustrations 12 and 13 show different expressions of the same character by way of example. The illustration 12 shows a smiling face and illustration 13 shows an angry face. Here an illustration display portion comprises the illustration display sub-portions 12a, 13a, and 14a.

As a way of folding the disposable diaper with such illustrations positioned thereon, Figs. 3C through 3H show an example of the way of folding the disposable diaper such that it can be contained in a compact bag and/or to be displayed for sale. First, side flap portions on the left and right sides of the ventral side and dorsal side of the disposable diaper are folded backwards along creases 32 and 33 as shown in Fig. 3A. Fig. 3D shows the ventral side of the disposable diaper in the state that the side flap portions have been folded. Fig. 3C is a plan view of this disposable diaper and shows the waist opening 2. Fig. 3E shows a side view of this disposable diaper.

Figs. 3F through 3H show three views of the disposable diaper in Fig. 3D having been folded into three along creases 34 and 35 so that the ventral side is now on the outer side. Fig. 3F shows an front illustration display sub-portion 12a of the disposable diaper in the folded state, Fig. 3G shows a rear illustration display sub-portion 13a of the disposable diaper in the folded state, and Fig. 3H shows a side view of the disposable diaper in the folded state. On the front illustration display sub-portion 12a, which is an outer face, illustration 12 positioned on the waist opening side of the ventral side is displayed in a non-divided manner or as a whole. And on the rear illustration display sub-portion 13a, which is the other outer face, the illustration 13 positioned on the groin portion side is also displayed in a non-divided manner.

The illustrations 12 and 13, which are positioned on the ventral side of the disposable diaper, are positioned such that the they do not overlap with the creases 32, 33, 34, or 35 when the disposable diaper is folded.

With the disposable diaper according to the embodiment shown in Figs. 3F and 3G, the illustrations 12 and 13 are displayed in a complete and independent manner on the two outer faces of the disposable diaper in the folded state. The main portions of illustrations 12 and 13 can thus be visually recognized even though the disposable diaper is in the folded state as shown in Figs. 3F and 3G.

With this disposable diaper, the diaper exchanger can show a story to the infant who is to be the wearer by using the illustrations on the disposable diaper and thereby creating an opportunity to communicate with the infant.

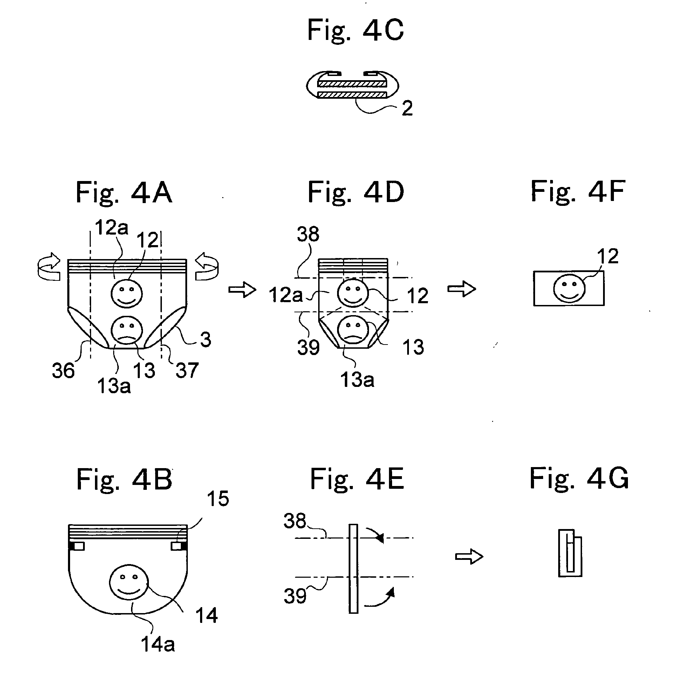

Figs. 4A through 4G show a disposable diaper with illustrations according to another embodiment of the present invention. Fig. 4A shows a ventral side of the disposable diaper and Fig. 4B shows a dorsal side of the disposable diaper. With the embodiment shown in Figs. 4A through 4G, though illustrations 12, 13, and 14 that are positioned at illustration display sub-portions 12a, 13a, and 14a are the same as those of the embodiment shown in Figs. 3A and 3B, waste disposing tapes 15 are positioned at two positions on the dorsal side of the disposable diaper, and these are located near the boundary of the dorsal side and the ventral side, and between each leg opening 3 and a waist opening 2. Here, an illustration display portion comprises illustration display sub-portions 12a, 13a, and 14a.

With the embodiment shown in Figs. 4A through 4G, first, side flap portions on the left and right sides of the ventral side and dorsal side of the disposable diaper are folded in the dorsal side direction along creases 36 and 37. Fig. 4D shows the ventral side of the disposable diaper in the state that the side flap portions are folded. Fig. 4C is a plan view of this disposable diaper and shows the waist opening 2. Fig. 4E shows a side view of this disposable diaper.

Figs. 4F and 4G show a subsequent state that the disposable diaper is further folded in three along creases 38 and 39 so that the ventral side becomes an outer face of the folded disposable diaper. Fig. 4F shows a front outer face of the disposable diaper in the folded state, and Fig. 4G shows a side view of the folded disposable diaper. The two illustrations 12, 13, which are positioned on the ventral side, are displayed in a non-divided manner on respective outer faces after the disposable diaper is folded, too.

The illustrations 12 and 13, which are positioned at the illustration display sub-portions 12a and 13a on the ventral side of the disposable diaper, are positioned such that they do not overlap with the creases 36, 37, 38, and 39 when the disposable diaper is folded. Thus, as shown in Fig. 4F, the main portion of the illustration 12 can be visually recognized even thought the disposable diaper is in the folded state.

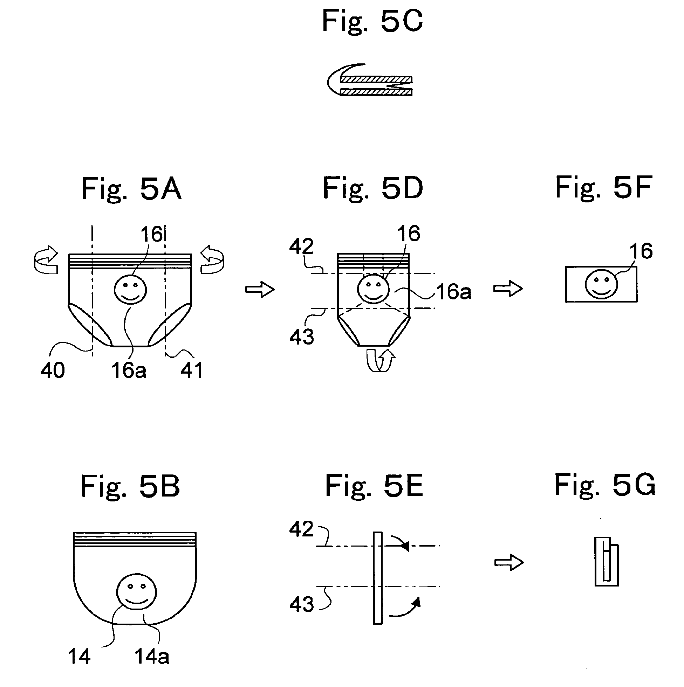

Figs. 5A through 5G show an disposable diaper with illustrations according to yet another embodiment of the present invention. Fig. 5A shows a ventral side of the disposable diaper and Fig. 5B shows a dorsal side of the disposable diaper. With the embodiment shown in Figs. 5A through 5G, one illustration 16 is positioned at a illustration display sub-portion 16a on the ventral side. Also, an illustration 14 is positioned at a illustration display sub-portion 14a on the dorsal side. Here, an illustration display portion comprises the illustration display sub-portions 14a and 16a.

With regard to the folding way, one side flap portion on the left side of the ventral side and the dorsal side is folded in the dorsal side direction along a crease 40 and the other side flap portion on the right side of the ventral side and the dorsal side is folded along a crease 41 towards the inner of the disposable diaper. Fig. 5D shows the ventral side of the disposable diaper in the state that the side flap portions are folded as described above. Fig. 5C is a plan view of this disposable diaper, which shows a waist opening 2. Fig. 5E shows a side view of this disposable diaper.

Next, Figs. 5F and 5G show a state that the diaper is further folded in three along creases 42 and 43 so that the ventral side becomes an outer face of the folded disposable diaper. Fig. 5F shows a front outer face of the folded disposable diaper and Fig. 5G is a side view of the folded disposable diaper. As with the other cases, the illustration 16, which is positioned at the illustration display sub-portion 16a on the ventral side, also appears without being divided by the creases 42, 43 after it is folded.

The illustration 16, which is positioned at the illustration display sub-portion 16a on the ventral side of the disposable diaper, is positioned such that that it does not overlap with the creases 40, 41, 42, or 43 when the disposable diaper is folded. Thus, as shown in Fig. 5F, the main portion of the illustration 16 can be visually recognized even though the disposable diaper is in the folded state.

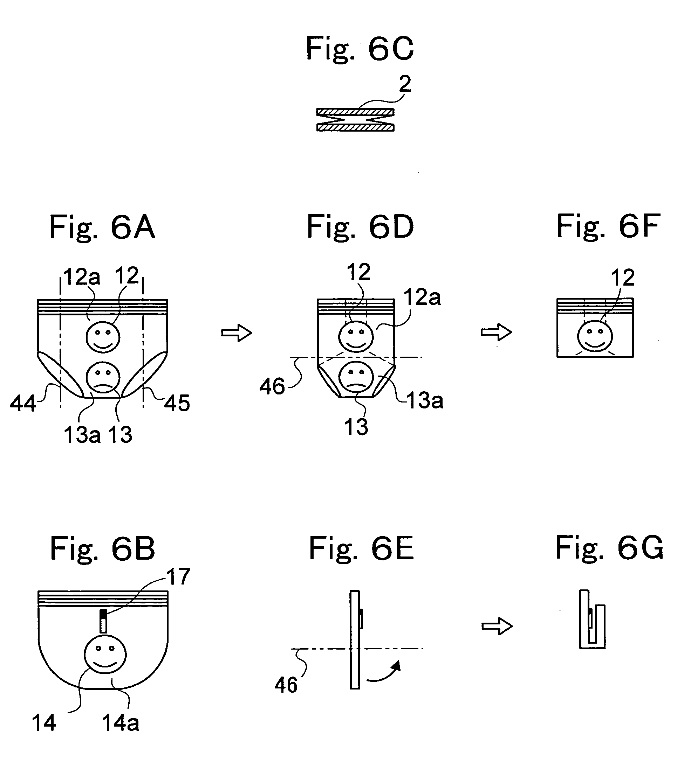

Figs. 6A through 6G show an disposable diaper with illustrations according to another embodiment of the present invention. Fig. 6A shows a ventral side of the disposable diaper and Fig. 6B shows a dorsal side of the disposable diaper. With the embodiment shown in Figs. 6A through 6G, two illustrations 12 and 13 are positioned at illustration display sub-portions 12a and 13a on the ventral side. Also, an illustration 14 is positioned at a illustration display sub-portion 14a on the dorsal side. Here, an illustration display portion comprises the illustration display sub-portions 12a, 13a, and 14a.

With regard to the way of folding the disposable diaper, first, side flap portions at the left and right sides of the ventral side and the dorsal side are folded towards the inner of the disposable diaper along creases 44 and 45. Fig. 6D shows the ventral side of the disposable diaper in the state that the side flap portions are folded. Fig. 6C is a plan view of this disposable diaper, which shows a waist opening 2. Fig. 6E is a side view of this disposable diaper.

Next, Figs. 6F and 6G show a state that the disposable diaper is folded further in two along a crease 46 so that the ventral side is become a rear outer face of the folded disposable diaper. Fig. 6F shows a front outer face of the folded disposable diaper and Fig. 6G is a side view of the folded disposable diaper. As with the other cases, the illustrations 12, 13, which are positioned at the illustration display sub-portions 12a, 13a on the ventral side, also appear without being divided by the crease 46 after the disposable diaper is folded.

The illustrations 12 and 13, which are positioned at the illustration display sub-portions 12a and 13a on the ventral side of the disposable diaper, are positioned such that they do not overlap with the creases 44, 45, or 46 when the disposable diaper is folded. Thus, as shown in Fig. 6F, the main portion of the illustration 12 can be visually recognized even though the disposable diaper is in the folded state.

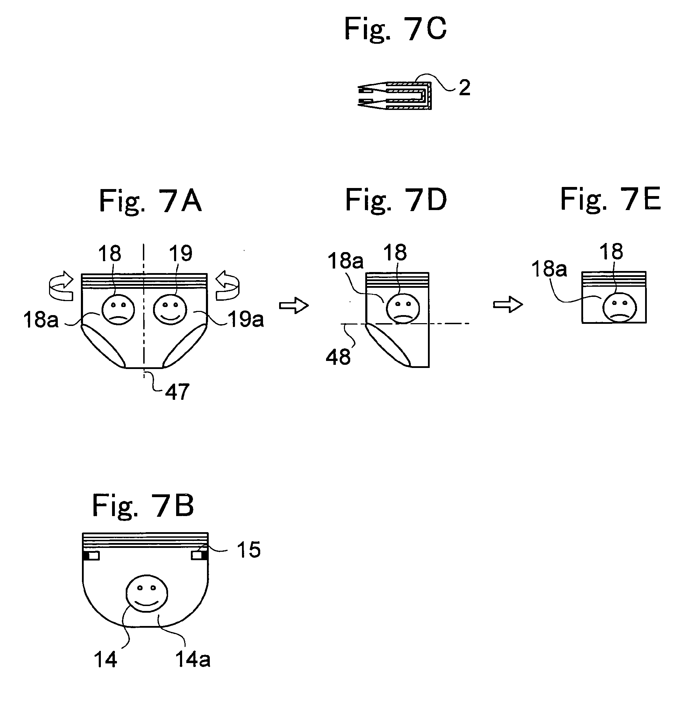

Figs. 7A through 7E show a disposable diaper with illustrations according to another embodiment of the present invention. Fig. 7A shows a ventral side of the disposable diaper and Fig. 7B shows a dorsal side of the disposable diaper. With the embodiment shown in Figs. 7A through 7E, though two illustrations 18 and 19 are positioned at illustration display sub-portions 18a and 19a on the ventral side, these two illustrations 18, 19 are positioned side by side to be aligned parallel to the edge line of the disposable diaper, along which a waist opening 2 is provided, and are placed separately on left and right sides, each of which has each leg opening. Also, an illustration 14 is positioned at a illustration display sub-portion 14a on the dorsal side. Here, an illustration display portion comprises the illustration display sub-portions 14a, 18a, and 19a.

With regard to the way of folding the disposable diaper, the disposable diaper is folded along a crease 47, which is positioned equidistantly from the two leg openings, so that the two leg openings overlap each other and the ventral side becomes outer faces of the folded disposable diaper. Fig. 7D shows the left ventral side of the disposable diaper in the state that the disposable diaper is folded along the crease 47. Fig. 7C is a plan view of the disposable diaper in the state that it is folded along the crease 47, which shows a waist opening 2.

If the disposable diaper is to be folded evenmore compactly, the disposable diaper is folded along a crease 48, which is a crease perpendicular to the other crease 47 and passes near the center of the disposable diaper. Fig. 7E shows the ventral side of the disposable diaper in the state that it is folded along the crease 48. On the two outer faces after folding, the two illustrations 18, 19, which are positioned at the illustration display sub-portions on the ventral side, appear respectively without being divided by the creases 47, 48.

The illustrations 18 and 19, which are positioned at the illustration display sub-portions 18a and 19a on the ventral side of the disposable diaper, are positioned such that they do not overlap with the creases 47, 48 when the disposable diaper is folded. Thus, as shown in Fig. 7E, the main portion of illustration 18 can be visually recognized even though the disposable diaper is in the folded state.

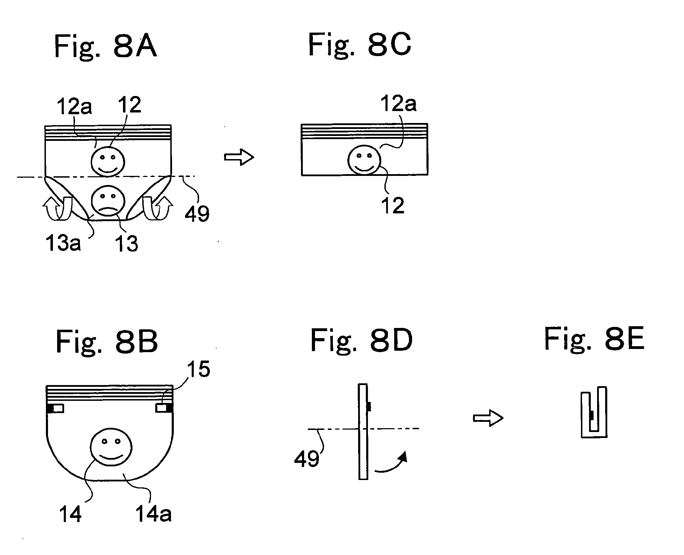

Figs. 8A through 8E show a disposable diaper with illustrations according to another embodiment of the present invention. Fig. 8A shows a ventral side of the disposable diaper, Fig. 8B shows a dorsal side of the disposable diaper, and Fig. 8D is a side view of the disposable diaper. With the embodiment shown in Figs. 8A through 8E, two illustrations 12 and 13, positioned at illustration display sub-portions 12a and 13a on the ventral side, are located on the waist opening side and the groin portion side, respectively. Also, an illustration 14 is positioned at a illustration display sub-portion 14a on the dorsal side. Here, an illustration display portion comprises the illustration display sub-portions 12a, 13a, and 14a.

With regard to the way of folding the disposable diaper, the disposable diaper is folded in two along a crease 49, which is parallel to an opening edge of the disposable diaper provided around a waist opening and near the center of the disposable diaper so that the ventral side becomes an outer face. Fig. 8C shows the ventral side of the disposable diaper in the state that it is folded along the crease 49. Fig. 8E is a side view of the disposable diaper in the state that it is folded along the crease 49. Each of the two illustrations 12, 13, which are positioned at the illustration display sub-portions 12a, 13a on the ventral side, appears without being divided by the crease 49 after folding.

The illustrations 12 and 13, which are positioned on the ventral side of the disposable diaper, are positioned such that they do not overlap with the crease 49 when the disposable diaper is folded. Thus, as shown in Fig. 8C, the main portion of illustration 12 can be visually recognized even though the disposable diaper is in the folded state.

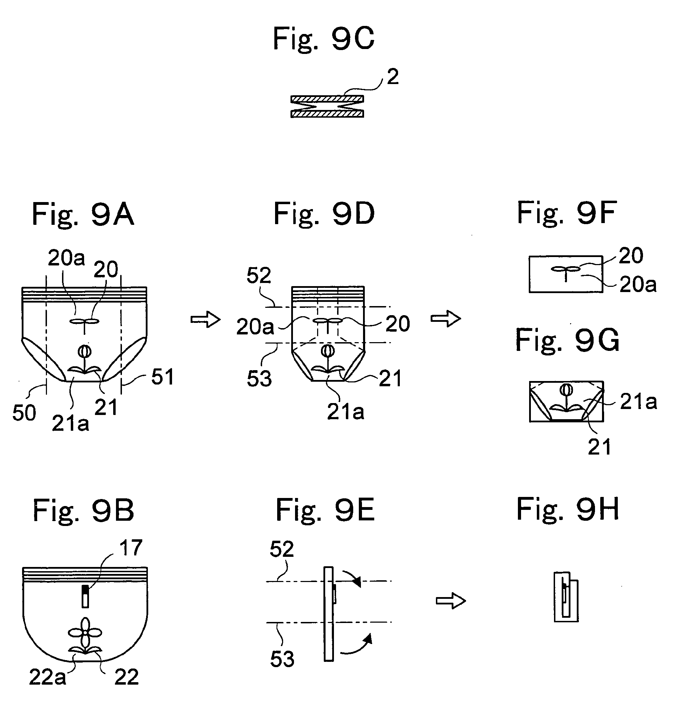

Figs. 9A through 9H show a disposable diaper with illustrations according to another embodiment of the present invention. Fig. 9A shows a ventral side of the disposable diaper and Fig. 9B shows a dorsal side of the disposable diaper. With the embodiment shown in Figs. 9A through 9H, the positioning of illustrations 20, 21, 22 and the way of folding the disposable diaper are the same as those of the diaper shown in Figs. 3A through 3H. However, this disposable diaper differs in that two illustrations 20 and 21 being positioned at illustration display sub-portions 20a and 21a on the ventral side, and an illustration 22 being positioned at an illustration display sub-portion 22a, respectively show changes with time of a flower, which is a character figure. For example, the illustration 20 shows the appearance of a sprout, the illustration 21 shows the growth of a bud, and the illustration 22 shows the blooming of a flower. Here, an illustration display portion comprises the illustration display sub-portions 20a, 21a, and 22a.

When the disposable diaper is folded along creases 50 and 51 according to this embodiment in the same manner as the embodiment shown in Figs. 3D and 3E, each of the two illustrations 20 and 21, which are positioned at illustration display sub-portions 20a and 21a on the ventral side, appears without being divided by the creases 52, 53 on outer faces of the disposable diaper after folding. Fig. 9D shows the ventral side of the disposable diaper in the state that the side flap portions are folded. Fig. 9C is a plan view of this disposable diaper, which shows a waist opening 2. Fig. 9E is a side view of this disposable diaper.

Figs. 9F through 9H show the disposable diaper is folded in three along the creases 52 and 53 so that the ventral side becomes outer faces. Fig. 9F shows a front outer face of the folded disposable diaper, Fig. 9G shows a rear outer face of the folded disposable diaper, and Fig. 9H is a side view of the folded disposable diaper.

The illustrations 20 and 21, which are positioned at the illustration display sub-portions 20a and 21a on the ventral side of the disposable diaper, are positioned such that they do not overlap with the creases 50, 51, 52, and 53 when the disposable diaper is folded. The main portions of illustrations 20 and 21 can thus be visually recognized even though the disposable diaper is folded as shown in Figs. 9F and 9G.

In the disposable diaper according to this embodiment, the illustration 20 of the first character figure in the time-line (That is to say, the illustration 20 corresponds to the appearance of a sprout.) can be shown first with the disposable diaper in the folded state.

The folded disposable diaper is then turned over to make the illustration 21, which shows the second character figure in the time-line (That is to say, the illustration 21 corresponds to the growth of a bud.), appear. The folded disposable diaper is then unfolded to make the illustration 22, which is positioned on the dorsal side and shows the third character figure in the time-line (That is to say, the illustration 22 corresponds to the blooming of a flower.), appear. The diaper exchanger can thus tell a complex story using the illustrations on the disposable diaper to the infant who is the wearer. An opportunity for communications especially with the infant, who is several months old and is in a stage of developing intellectual faculties, is thus provided.

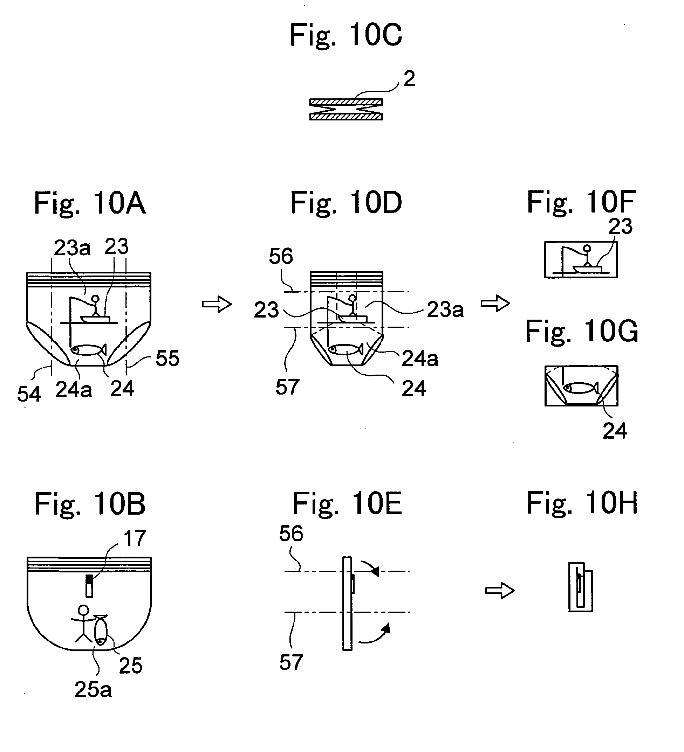

Figs. 10A through 10H show a disposable diaper with illustrations according to another embodiment of the present invention. Fig. 10A shows a ventral side of the disposable diaper and Fig. 10B shows a dorsal side of the disposable diaper. With the embodiment shown in Figs. 10A through 10H, the positioning of the illustrations 23, 24, 25 and the way of folding the disposable diaper are the same as those of the disposable diaper shown in Figs. 3A through 3H. Two illustrations 23 and 24, positioned at illustration display sub-portions 23a and 24a on the ventral side, and an illustration 25, positioned at a illustration display sub-portion 25a, express a story containing different characters. And the illustrations 23, 24, 25 show changes with time or location and a logical development. For example, the illustration 23 expresses a person dangling a fishing line, the illustration 24 expresses a swimming fish, and the illustration 25 expresses the person with the fish in his hand. Here, an illustration display portion comprises the illustration display sub-portions 23a, 24a, and 25a.

When the disposable diaper according to the present embodiment is folded along creases 54 and 55 in the same manner as the embodiment shown in Figs. 9D and 9E, each of the two illustrations 23 and 24, which are positioned at illustration display sub-portions 23a and 24a on the ventral side, appears without being divided by the creases on each of the outer faces after folding as shown in Figs. 10F and 10G. Fig. 10D shows the ventral side of the disposable diaper in the state that the side flap portions are folded. Fig. 10C is a plan view of this disposable diaper, which shows a waist opening 2. Fig. 10E is a side view of this disposable diaper.

Figs. 10F through 10H show how the disposable diaper with illustrations is folded in three along creases 56 and 57 so that the ventral side becomes outer faces of the folded disposed diaper. Fig. 10F shows a front outer face of the folded disposable diaper, Fig. 10G shows a rear outer face of the folded disposable diaper, and Fig. 10H is a side view of the folded disposable diaper.

The illustrations 23 and 24, which are positioned at the illustration display sub-portions 23a and 24a on the ventral side of the disposable diaper, are positioned such that they do not overlap with the creases 54, 55, 56, or 57 when the disposable diaper is folded. The main portions of the illustrations 23 and 24 can thus be visually recognized even though the disposable diaper is in the folded state. Here, the main portions of the illustrations 23 and 24 are the person dangling a fishing line and the swimming fish. Therefore, the fishing line itself is not a main portion of either illustration.

With the disposable diaper according to this embodiment, the illustration 23 of the character figure that is the leading character in the development of a story (That is to say, the illustration 23 corresponds to the person dangling a fishing line.) can be shown first with the disposable diaper in the folded state. The folded disposable diaper is then turned over to make the illustration 24 of the character figure that is the supporting character of the story (That is to say, the illustration 24 corresponds to the swimming fish.) appear. The folded disposable diaper is then unfolded to show a first entire scene of the leading and supporting characters for the development of the story (the integrated illustration of the illustrations 23 and 24 showing the person dangling a fishing line above the swimming fish), which is positioned on the ventral side. Lastly, the conclusion of the story of the person with the fish in his hand, which is positioned on the dorsal side, is shown. The diaper exchanger can thus show an even more complex story using the disposable diaper with illustrations to the infant who is the wearer. An opportunity for communications especially with the infant, who is several months old and is in a stage of developing intellectual faculties, is thus provided.

With the disposable diaper with illustrations according to the present invention, since the illustrations can be recognized clearly from the outside even though the disposable diaper is in the folded state, the aesthetic appearances of the illustrations is not damaged. Furthermore, the illustrations can be used to create an opportunity for communications between the diaper exchanger and the infant from when the disposable diaper is in the folded state and the mutual relationship between the exchanger and the infant thus becomes good.

Furthermore, since the illustrations can be shown on a plurality of the illustration display sub-portions and the story can be developed with the illustrations that are respectively shown on the plurality of illustration display sub-portions, the mutual communications between the infant and the diaper exchanger can be even increased.

The infant can thus be interested in the illustrations of the disposable diaper without any toys or other objects before and during the disposable diaper exchanging period. Furthermore, by deepening the relationship between the exchanger and the infant, the infant would not be fussy about exchanging diapers. And the exchange of the disposable diaper can thus be carried out smoothly and pleasantly.

Also as another achievement, the lowering of a function such as decrease in the adhesive strength of the waste disposal means may be effectively prevented and the occurrence of defects during the manufacture can be reduced.

## Claims

1. A disposable diaper with illustrations comprising an illustration display portion;
wherein the illustration display portion comprises a plurality of illustration display sub-portions on which the illustrations are displayed ;
wherein the illustration display sub-portions are provided on a plurality of display faces of the disposable diaper respectively; and
wherein at least a main portion of one of the illustrations is provided at a position such that the main portion is visually recognizable from an outside when the disposable diaper is folded in a way of folding so as to be contained in a compact bag and/or to be displayed for sale.

2. A disposable diaper with illustrations comprising an illustration display portion;
wherein the illustration display portion comprises a plurality of illustration display sub-portions on which different illustrations are displayed, respectively;
wherein the illustration display sub-portions are respectively provided at a plurality of display faces of the disposable diaper; and
wherein at least one illustration among the illustrations is not divided by being folded, the illustrations being provided at visually recognizable positions from an outside when the disposable diaper is folded in the way of folding so as to be contained in a compact bag and/or to be displayed for sale.

3. The disposable diaper according to Claim 2, wherein none of the illustrations is divided by being folded, the illustrations being provided at visually recognizable positions from an outside when the disposable diaper is folded in the way of folding so as to be contained in a compact bag and/or to be displayed for sale.

4. The disposable diaper according to any one of Claims 1 to 3, wherein the disposable diaper is folded in a way of folding such that first and second illustrations among the illustrations such that the folded disposable diaper has two outside faces and the first and second illustrations are provided on the two outside faces, respectively.

5. The disposable diaper according to any one of Claims 1 to 4, further comprising another illustration provided at an inside face of folded portions of the disposable diaper being provided when the disposable diaper is folded in the way of folding so as to be contained in a compact bag and/or to be displayed for sale.

6. The disposable diaper according to any one of Claims 1 to 5, wherein yet another illustration among the illustrations is completed within any one of outside faces of the folded disposable diaper, the yet another illustration being provided at a visually recognizable position from an outside when the disposable diaper is folded in the way of folding so as to be contained in a compact bag and/or to be displayed for sale.

7. The disposable diaper according to any one of Claims 1 to 6, wherein each illustration among the illustrations has a feature of a story, the each illustration being provided at respective outside faces of the disposable diaper when the disposable diaper is folded in the way of folding so as to be contained in a compact bag and/or to be displayed for sale.

8. The disposable diaper according to any one of Claims 1 to 7, comprisingmeans for disposing provided on an outer surface of the disposable diaper, wherein the disposable diaper is folded such that the means for disposing is hidden when the disposable diaper is folded in the way of folding so as to be contained in a compact bag and/or to be displayed for sale.

9. The disposable diaper according to Claim 8, wherein the means for disposing is provided off a crease of the disposable diaper and the illustrations.

10. The disposable diaper according to Claim 8 or 9, wherein the means for disposing comprises a folded tape.

11. The disposable diaper according to any one of Claims 1 to 10, wherein the disposable diaper is formed as a pants type diaper.

12. The disposable diaper according to any one of Claims 1 to 11, comprising a ventral portion that is positioned at a ventral side of a wearer when worn, a dorsal portion that is positioned at a dorsal side of the wearer when worn, a pair of left and right leg openings at the side edges of the ventral and dorsal portions, and a waist opening at the upper edges of the ventral and dorsal portions.

13. A disposable diaper with an illustration comprising a crease along which the disposable diaper is folded, wherein the illustration is provided off the creases.

14. The disposable diaper according to Claim 13, wherein the illustration and the crease are provided in a substantially not overlapping manner.
